(19) Europäisches Patentamt
European Patent Office
Office européen des brevets

(11) Veröffentlichungsnummer: **0 002 518**
**B1**

(12) # EUROPÄISCHE PATENTSCHRIFT

(45) Veröffentlichungstag der Patentschrift: **04.11.81**

(51) Int. Cl.³: **C 07 D 223/10,** C 08 L 61/32, D 21 H 3/52, C 08 G 12/40

(21) Anmeldenummer: **78101646.4**

(22) Anmeldetag: **12.12.78**

(54) **Kondensationsprodukt aus Epsilon-Caprolactam, Formaldehyd und Formamid, mit diesem Kondensationsprodukt modifizierter Aminoplast und Verfahren zur Herstellung des Aminoplasts.**

(30) Priorität: **14.12.77 DE 2755589**
**14.12.77 DE 2755588**

(43) Veröffentlichungstag der Anmeldung: **27.06.79 Patentblatt 79/13**

(45) Bekanntmachung des Hinweises auf die Patenterteilung: **04.11.81 Patentblatt 81/44**

(84) Benannte Vertragsstaaten: **BE CH DE FR GB IT NL SE**

(56) Entgegenhaltungen:
**DE-A-2 049 378**
**FR-A-2 176 717**

(73) Patentinhaber: **CASSELLA Aktiengesellschaft, Hanauer Landstrasse 526, D-6000 Frankfurt am Main 61 (DE)**

(72) Erfinder: **Piesch, Steffen, Dr., An der Heide 32, D-6370 Oberursel (DE)**
Erfinder: **Dörries, Peter, Hansa-Allee 80, D-6000 Frankfurt am Main 1 (DE)**
Erfinder: **Wolf, Alfons, Einhardstrasse 34, D-6453 Seligenstadt (DE)**
Erfinder: **Wille, Herbert, Dr., Hünfelder Strasse 16, D-6000 Frankfurt am Main 61 (DE)**

(74) Vertreter: **Urbach, Hans-Georg, Dr. et al, Hanauer Landstrasse 526, D-6000 Frankfurt am Main 61 (DE)**

EP 0 002 518 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Kondensationsprodukt aus ε-Caprolactam, Formaldehyd und Formamid, mit diesem Kondensationsprodukt modifizierter Aminoplast und Verfahren zur Herstellung des Aminoplasts

Die Erfindung betrifft ein Kondensationsprodukt aus ε-Caprolactam, Formaldehyd und Formamid im Molverhältnis 1 : a : b, wobei a eine Zahl von 1 bis 20, b eine Zahl von 1 bis 19 bedeutet und a und b so gewählt werden, daß der Quotient

$$\frac{a}{b+1} = 0{,}5 \text{ bis } 1$$

darstellt, insbesondere das N-(Formylaminomethyl)-ε-caprolactam und das N-(N'-Formyl-N'-hydroxymethyl-aminomethyl)-ε-caprolactam.

Ferner betrifft die Erfindung einen Aminoplast, der das Kondensationsprodukt aus ε-Caprolactam, Formaldehyd und Formamid als Modifizierungskomponenten enthält, und ein Verfahren zu seiner Herstellung.

Der modifizierte Aminoplast ist hervorragend als Tränkharz für die Tränkung von Papier- und Gewebebahnen, die zur Herstellung beschichteter Holzwerkstoffe und Schichtstoffe verwendet werden, geeignet.

Das erfindungsgemäße Kondensationsprodukt aus ε-Caprolactam, Formaldehyd und Formamid im Molverhältnis 1 : a : b, wobei a eine Zahl von 1 bis 20, b eine Zahl von 1 bis 19 bedeutet und a und b so gewählt werden, daß der Quotient

$$\frac{a}{b+1} = 0{,}5 \text{ bis } 1$$

darstellt, wobei a und b nicht notwendigerweise ganzzahlig sein müssen, wird durch Erhitzen von Formamid und Formaldehyd in Gegenwart einer Base und gegebenenfalls von ε-Caprolactam bei Temperaturen von 70 bis 90°C während 4 bis 15 Stunden, Sauerstellen des Reaktionsansatzes und, sofern noch kein ε-Caprolactam vorhanden ist, Hinzufügen von ε-Caprolactam und Abdestillieren von Wasser bei Temperaturen von 100 bis 135°C hergestellt. Als Base wird normalerweise ein Alkalihydroxid oder Alkalicarbonat verwendet und die Reaktion zweckmäßigerweise unter Rühren durchgeführt. Zum Sauerstellen des Reaktionsgemisches wird beispielsweise Kaliumhydrogensulfat zugegeben. Zweckmäßigerweise wird das bei der Kondensation entstehende Wasser azeotrop und/oder unter vermindertem Druck abdestilliert. Für die azeotrope Destillation ist die Zugabe eines Schleppmittels, wie z. B. Toluol, zu dem Ansatz erforderlich. Für die Destillation unter vermindertem Druck können beispielsweise Drucke von 10 bis 500 mbar, insbesondere 100 bis 350 mbar, zur Anwendung kommen. Es ist vorteilhaft, einen solchen Unterdruck zu wählen, daß die Temperatur der Reaktionsmischung 135°C nicht übersteigt. Wenn das ε-Caprolactam bereits zu Beginn der Reaktion zusammen mit Formaldehyd und Formamid eingesetzt wird, tritt es erst nach dem Sauerstellen des Ansatzes in die Reaktion ein.

Der benötigte Formaldehyd wird zweckmäßigerweise in wasserfreier Form, d. h. beispielsweise als Paraformaldehyd (Polyoxymethylen) oder Trioxan eingesetzt. Es ist auch möglich, gasförmigen Formaldehyd zu verwenden. Auch möglichst konzentrierte Lösungen von Formaldehyd in organischen Lösungsmitteln, die sich bei der Reaktion inert verhalten, oder in Wasser können verwendet werden. Die Verwendung von Lösungen erfordert jedoch die spätere Abdestillation des Lösungsmittels.

Zur Herstellung des N-(Formylaminomethyl)-ε-caprolactams der Formal Ia

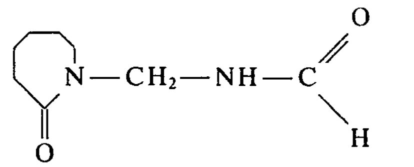

(Ia)

werden die Ausgangsverbindungen im Molverhältnis ε-Caprolactam zu Formaldehyd zu Formamid = 1 : 1 : 1 eingesetzt. Zur Herstellung des N-(N'-Formyl-N'-hydroxymethylaminomethyl)-ε-caprolactams der Formel Ib

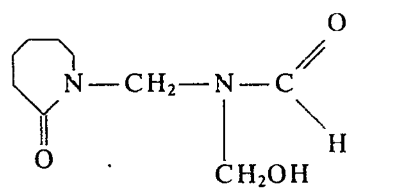

(Ib)

werden die Ausgangsverbindungen im Molverhältnis ε-Caprolactam zu Formaldehyd zu Formamid = 1 : 2 : 1 eingesetzt.

Nach der Abdestillation des bei den Reaktionen entstehenden Wassers wird ein allenfalls vorhandenes Schleppmittel abdestilliert. Man erhält in praktisch quantitativer Ausbeute die Kondensationsprodukte in Form farbloser Öle, die nicht kristallisieren und die in Wasser löslich sind. Die Verbindung der Formel Ia kann durch Vakuumdestillation gereinigt werden. Die Verbindung Ib kann auch durch Methylolierung des N-(Formylaminomethyl)-ε-caprolactams der Formel Ia sowie durch Umsetzung von Bis(hydroxymethyl)-formamid mit ε-Caprolactam hergestellt werden.

Die erfindungsgemäßen Kondensationsprodukte, die Verbindungen der Formel Ia und Ib, können auch in Form ihrer Rohprodukte und Gemische sowie ihrer wäßrigen Lösungen zur Modifizierung von Aminoplasten verwendet werden.

Aminoplaste sind harzartige Produkte und deren Lösungen, die durch Kondensation von Amino- oder Iminogruppen enthaltenden Verbindungen, den sogenannten Aminoplastbildnern und Carbonylverbindungen und gegebenenfalls einem niederen Alkanol entstehen. Die Reaktion zwischen den Aminoplastbildnern, den Carbonylverbindungen und gegebenenfalls dem Alkanol wird dabei soweit durchgeführt, daß die Produkte noch löslich und schmelzbar bleiben. Sobald dieser Zustand erreicht ist, wird die Kondensation abgebrochen, z. B. durch Abkühlen und Einstellen eines schwach alkalischen pH-Wertes der Reaktionsmischung. Die so hergestellten, nicht auskondensierten Aminoplaste (auch Aminoplastvorkondensate genannt) werden in Form ihrer wäßrigen Lösungen, insbesonderer als Tränkharze für die Schichtpreßstoff-Industrie und zur Oberflächenveredelung von Holzwerkstoffen benutzt.

Bei der Oberflächenveredelung von Holzwerkstoffen wird auf Holzfaser- oder Holzspanplatten eine Dekor- bzw. Schutzschicht dadurch aufgebracht, daß dekorative Papier- bzw. Gewebebahnen mit geeigneten Aminoplasten, vorzugsweise Melaminharzen, getränkt und mit einem bestimmten Restfeuchtegehalt auf die Platten des Holzwerkstoffs durch Thermohärtung auflaminiert werden. Der Preßdruck kann dabei etwa 10 bis 100 bar (1 bar = $10^5$Pa $\approx$ 1 kg/cm$^2$) und die Temperatur 120 bis 180°C betragen. Während des Preßvorgangs härtet der Aminoplast aus und verbindet die Papier- bzw. Gewebebahn mit der Platte des Holzwerkstoffs. In ähnlicher Weise lassen sich Schichtstoffe mit dekorativer bzw. schützender Oberfläche herstellen. Hierbei wird die aminoplastharzgetränkte Dekorbahn auf mehrere Lagen phenolharzimprägnierter Kraftpapiere durch Thermohärtung aufgepreßt. Die Preßdrucke liegen hierbei im Bereich von ca. 50 bis 150 bar, während die Preßtemperaturen wie bei der dekorativen Beschichtung vor Holzwerkstoffen etwa 120 bis 180°C betragen. Zur Erhöhung der Kratz- und Abriebfestigkeit wird vor dem Verpressen auf die Dekorbahn ein aminoplastharzgetränktes transparentes, sogenanntes Overlay-Papier aufgelegt. Zwischen Dekorbahn und Kern kommt manchmal noch ein Barrierepapier und auf der Rückseite ein Gegenzugpapier zur Anwendung. Für die Tränkung der Overlay- und der Dekorbahn kommen vorzugsweise Melamin-Formaldehyd-Harze zur Anwendung. Auch bei der Oberflächenveredelung von Holzwerkstoffen können Overlay- und Barrierepapiere zur Anwendung kommen.

Beschichtete Holzwerkstoffe und Schichtstoffe zeichnen sich durch ihre guten chemischen und physikalischen Eigenschaften aus. Die zum Tränken der für die Dekor- bzw. Schutzschicht vorgesehenen Papier- bzw. Gewebebahnen benutzten, nicht elastifizierten Aminoplaste besitzen jedoch im ausgehärteten Zustand nur eine geringe Elastizität, weshalb mit diesen Harzen hergestellte Oberflächen zur Rißbildung neigen.

Es hat nicht an Versuchen gefehlt, die mangelnde Elastizität der Dekorschicht durch Zusätze zu den Tränkharzen zu beseitigen oder zu verringern. Insbesondere wurden als Zusätze Polyalkohole, Sorbit und Zucker, sowie aromatische Sulfonamide empfohlen. Beim Zusatz von Polyalkoholen oder Zuckern wird jedoch die Wasserfestigkeit der ausgehärteten Harze ungenügend, wenn die Elastizität ausreichend sein soll. Sulfonamide allein reichen zur Erzielung einer guten Elastizität nicht aus.

Bei der Verarbeitung der Aminoplaste erfolgt ein Übergang der löslichen und schmelzbaren Aminplastvorkondensate in unschmelzbare und unlösliche Produkte. Bei dieser Aushärtung tritt eine Vernetzung ein. Die Geschwindigkeit dieser Vernetzungsreaktion ist jedoch auch bei den erhöhten Verarbeitungstemperaturen für anwendungstechnische Prozesse zu gering und muß daher durch Zusätze von sogenannten Härtern beschleunigt werden. Als Härter werden sauer reagierende und/oder säureabspaltende Verbindungen verwendet. Derartige Härter sind beispielsweise Ammonium- oder Aminsalze, z. B. Ammoniumchlorid, Ammoniumrhodanid, Äthanolaminhydrochlorid oder starke organische Säuren, wie z. B. p-Toluolsulfonsäure. Bei der Verwendung freier Säuren oder stark sauer reagierender Salze ergeben sich für das Aminoplastharz relativ geringe Topfzeiten, wodurch die Verarbeitung beeinträchtigt wird.

Bei einem Zusatz von Caprolactam als Modifizierungsmittel für die Erhöhung der Elastizität der fertigen Oberflächen können den Tränkharzen verhältnismäßig hohe Härtermengen zugefügt werden und dabei trotzdem eine hinreichend lange Gebrauchsdauer vor der Verwendung erzielt werden. Unbefriedigend ist jedoch die nicht sehr gute Elastizitätsreserve, die verminderte Wasserfestigkeit und ein ungleichmäßiger Glanz der damit hergestellten Oberflächen.

Tränkharze, die sich für den Zusatz großer Härtermengen oder härtungsbeschleunigender Zusätze und damit für die besonders rasche Aushärtung bzw. Verarbeitung eignen, werden im weitesten Sinne des Wortes als »Kurztaktharze« bezeichnet und haben sich in kurzer Zeit einen großen Markt erobert.

Es ist bekannt (DE-A-2 149 970), daß die Rißbildung in der Oberfläche von Holzwerkstoffen und Schichtpreßstoffen beseitigt bzw. ihre Elastizität erhöht werden kann, wenn die für die Dekor- bzw. Schutzschicht vorgesehenen Papier- bzw. Gewebebahnen mit einem mit Methylenbisformamid modifizierten Aminoplastharz getränkt und anschließend in an sich bekannter Weise auf Holzwerkstoffplatten auflaminiert oder zu einem Schichtpreßstoff verarbeitet werden. Auch bei der Verwendung methylenbisformamidmodifizierter Aminoplastharze als sogenannte Kurztaktharze erfolgt die Ausbildung eines gleichmäßigeren Glanzes der vergüteten Oberflächen. In der Praxis hat sich jedoch gezeigt, daß bei einer längeren Lagerung methylenbisformamidmodifizierter Aminoplastharze ihre anwendungstechnischen Vorteile, insbesondere die Rißbeständigkeit, teilweise wieder verlorengehen.

Aus der DE-A-2 049 378 sind als Tränkharze

Melamin-Formaldehyd-Kondensationsprodukte bekannt, die, bezogen auf Trockensubstanz, 0,5 bis 20 Gew.-% eines N-Methylolacrylamids oder N-Methylolmethacrylamids als Modifizierungsmittel enthalten. Als Kondensationsprodukt aus Melamin und Formaldehyd kann dabei auch ein Kondensationsprodukt aus Melamin, Formaldehyd und ε-Caprolactam verwendet werden.

Aus der FR-A-2 176 717 ist ein Verfahren zur Herstellung von Melamin-Formaldehyd-Tränkharzen bekannt, bei dem vor, während oder nach der Kondensation zwischen Melamin und Formaldehyd ein Dialkylfettamid in Mengen von 1 bis 20 Gew.-%, bezogen auf Vorkondensat, als Modifizierungsmittel zugesetzt wird. Dabei wird auch erwähnt, daß gegebenenfalls weitere Modifizierungsmittel, z. B. Caprolactam oder Toluolsulfonamid in geringen Mengen zugesetzt werden können.

Der Stand der Technik gibt keine Hinweise auf die Erfindung.

Bei der Oberflächenveredelung von Holzwerkstoffen und der Herstellung von Schichtstoffen können einwandfreie Oberflächen unter Verwendung eines ausreichend lagerfähigen Aminoplastharzes erhalten werden, wenn die für die Dekor- bzw. Schutzschicht vorgesehene Papier- bzw. Gewebebahn mit einem Aminoplasten, der mit einer erfindungsgemäßen Verbindung modifiziert ist, getränkt und anschließend in an sich bekannter Weise auf die Holwerkstoffplatte auflaminiert oder zu einem Schichtstoff verarbeitet wird.

Der mit einer erfindungsgemäßen Verbindung modifizierte Aminoplast besitzt normalerweise, bezogen auf den Festkörpergehalt des fertigen Harzes, einen Gehalt von 0,5 bis 40 Gew.-%, vorzugsweise 2,5 bis 15 Gew.-% an einer Verbindung der Formel Ia oder Ib oder eines Kondensationsproduktes ε-Caprolactam zu Formaldehyd zu Formamid = 1 : a : b, wobei a eine Zahl von 1 bis 20 und b eine Zahl von 1 bis 19 darstellt und a und b so gewählt werden, daß der Quotient

$$\frac{a}{b+1} = 0{,}5 \text{ bis } 1$$

beträgt. Praktisch erfolgt die Modifizierung eines Aminoplasten mit einem erfindungsgemäßen Kondensationsprodukt so, daß ein Aminoplastbildner in an sich bekannter Weise mit einer Carbonylverbindung und gegebenenfalls weiteren Modifizierungsmitteln, wie wasserlöslichen Alkoholen, kondensiert und vor, oder während oder nach der Kondensation eine erfindungsgemäße Verbindung der Formel Ia oder Ib oder ein Kondensationsprodukt aus ε-Caprolactam, Formaldehyd und Formamid mit dem Molverhältnis ε-Caprolactam zu Formaldehyd zu Formamid = 1 : a : b, wobei a eine Zahl von 1 bis 20 und b eine Zahl von 1 bis 19 darstellt und a und b so gewählt werden, daß der Quotient

$$\frac{a}{b+1} = 0{,}5 \text{ bis } 1$$

beträgt, vorzugsweise 1 : (1 bis 2) : 1, hinzugefügt wird.

Als Ausgangsprodukte für die Herstellung der Aminoplaste kommen die bekannten Aminoplastbildner, wie z. B. Harnstoff, Thioharnstoff, Di-cyandiamid, Guanamine, wie Aceto- oder Benzoguanamin, insbesondere jedoch Melamin und die zur Kondensation mit Aminoplastbildnern, bekannten Carbonylverbindungen, das sind aliphatische und aromatische Aldehyde und Ketone, wie z. B. Acetaldehyd, Butyraldehyd, i-Butyraldehyd, Aceton, Methyläthylketon etc., insbesondere jedoch Formaldehyd, in Betracht. Auch Mischungen von Aminoplastbildnern und/oder von Carbonylverbindungen können zur Herstellung des Aminoplasts verwendet werden. Melamin-Formaldehyd-Kondensationsprodukte sind besonders bevorzugt, aber auch Mischkondensate von Formaldehyd, Melamin und anderen Aminoplastbildnern, insbesondere Harnstoff, sowie Mischungen von Melamin-Formaldehyd-Kondensaten und Kondensationsprodukten von Formaldehyd und anderen Aminoplastbildnern, insbesondere Harnstoff, haben sich zur Herstellung erfindungsgemäßer Harze besonders bewährt.

Das Modifizierungsmittel kann auch in Form wäßriger Lösungen zugefügt werden. Bei Verwendung eines Kondensationsproduktes ε-Caprolactam zu Formaldehyd zu Formamid = 1 : 1 : 1, bzw. der Verbindung Ia, erfolgt die Zugabe zweckmäßigerweise gegen Ende der Kondensation, am besten nach beendeter Kondensation zu der abgekühlten Harzlösung. Es ist auch möglich, einen Teil des Modifizierungsmittels bereits zu Beginn der Harzkondensation zuzufügen und den Rest während oder nach der Kondensation zuzugeben. Falls ein Kondensationsprodukt mit dem Molverhältnis ε-Caprolactam zu Formaldehyd zu Formamid = 1 : 2 : 1, bzw. die Verbindung Ib, verwendet wird, ist es zweckmäßig, zunächst das Kondensationsprodukt aus ε-Caprolactam, Formaldehyd und Formamid herzustellen und nach anschließender Zugabe des Aminoplastbildners und der Carbonylverbindung die Kondensation des Harzes vorzunehmen. Erfolgt die Zugabe des Kondensationsproduktes aus ε-Caprolactam zu Formaldehyd zu Formamid vor oder während der Kondensation des Harzes, dann reagiert normalerweise mindestens ein Teil mit den Harzbildnern.

Nach beendeter Aminoplastkondensation können den modifizierten Aminoplasten auch Härter bzw. Härtungsbeschleuniger, z. B. Salze schwacher bis starker organischer Säuren, beispielsweise Diäthanolaminoacetat, Äthanolaminhydrochlorid, Äthylendiaminacetat, Ammoniumrhodanid, Ammoniumlactat oder Äthylendiaminphosphat zugefügt werden, um die Härtung zu beschleunigen, ohne daß sich dadurch die Elastizität der Überzüge verschlechtert.

Bei der Herstellung der Harze können auch noch andere Modifizierungsmittel, wie wasserlösliche Mono- oder Dialkohole, z. B. Methanol, Äthanol, Äthylenglykol, Äthylendiglykol, ferner Pentaaerythrit, Carbamate, wie z. B. Methylcarbamat, Methoxyäthylcarbamat, Salze der Malein- oder Fumaramidsäure, Zucker, Sorbit, Salze der Amidosulfosäure, aromatische Sulfonsäureamide und dergleichen zugefügt werden.

Bei der Harzherstellung wird wie üblich die Kondensation nur soweit durchgeführt, daß die Harze noch löslich und schmelzbar bleiben. Dabei wird in der Regel bis zu einer begrenzten Wasserverdünnbarkeit kondensiert. In manchen Fällen, z. B. bei Zusatz von größeren Mengen von Salzen der Amidosulfosäure, können die erhaltenen Harze auch unbegrenzt wasserlöslich sein. Zur Bestimmung der Wasserverdünnbarkeit wird eine Probe des Harzes mit Wasser bei 20°C titiert. Zum Beispiel besagt die Angabe »Wasserverdünnbarkeit 1 : X", daß 1 ml Harz bei 20°C X ml Wasser aufnehmen kann, ohne daß eine Trübung auftritt. Hinweise auf die Durchführung der Kondensation bei der Aminoplastherstellung sind z. B. enthalten in Kirk-Othmer, Encyclopedia of Chemical Technology, 1. Auflage, Vol 1 (1947), 756—759; Houben-Weyl »Methoden der organischen Chemie«, Bd. XIV/2, »Makromolekulare Stoffe« Teil 2, 1963, Georg Thieme Verlag Stuttgart, insbesondere Seiten 346 bis 357 (Harnstoffkondensate), Seiten 357 bis 371 (Melaminkondensate), Seiten 382 bis 388 (Kondensationsprodukte von Dicyandiamide und Guanidin); John F. Blais »Amino Resins« Reinhold Publishing Corp., New York (1959), Seiten 26 bis 53; C. P. Vale »Aminoplastics« Cleaver Hume Press Ltd., London (1950), Seiten 12 bis 87; Ullmanns Encyklopädie der technischen Chemie, 4. Auflage, Band 7 (1973), Seiten 403 bis 414.

Die Herstellung von dekorativ beschichteten Holzwerkstoffplatten unter Verwendung der erfindungsgemäßen Aminoplaste erfolgt so, daß die Papier- bzw. Gewebebahn mit einem erfindungsgemäßen Aminoplasten getränkt und in an sich bekannter Weise weiterverarbeitet wird. Die getränkte und getrocknete Papier- bzw. Gewebebahn wird somit auf die vorbereitete Holzwerkstoffplatte unter Drucken von ca. 10 bis 100 bar und Temperaturen von ca. 120 bis 180°C aufgepreßt, wobei für die Pressung vorteilhafterweise Mehretagenpressen verwendet werden. Analog können beim Einsatz von mit Phenolharzen imprägnierten Trägerbahnen anstelle der Holzwerkstoffplatten Schichtstoffe hergestellt werden, wobei Preßdrucke von ca. 50 bis 150 bar und Temperaturen von ca. 120 bis 180°C zur Anwendung kommen.

Hinweise auf die Herstellung beschichteter Holzwerkstoffplatten und von Schichtstoffen finden sich in:

John F. Blais loc. cit., Seiten 122—138; C. P. Vale loc. cit., Seiten 209—214, und Ullmann loc. cit., Seiten 417—418.

Die unter Verwendung der mit den erfindungsgemäßen Kondensationsprodukten modifizierten Aminoplaste hergestellten Schichtstoffe und beschichteten Holzwerkstoffe zeigen hohen gleichmäßigen Glanz, einwandfreie Aushärtung und hohe Elastizität und erfüllen auch die übrigen, an eine einwandfreie Oberfläche gestellten Forderungen. Die Aminoplaste sind lagerbeständig, d. h. die hervorragenden, bei der Verarbeitung der Aminoplaste auftretenden Ergebnisse werden während der gesamten Lebenszeit des Harzes erzielt. Besonders gute Ergebnisse werden bei Verwendung der Verbindung der Formel Ia zur Herstellung der Aminoplaste erreicht. Auch bei Verwendung der Verbindung der Formel Ib werden sehr gute Ergebnisse erzielt. Die Verbindungen der Formel Ia und Ib können daher auch in unreiner Form oder in Form von Gemischen, beispielsweise in Form eines Kondensationsproduktes aus ε-Caprolactam zu Formaldehyd zu Formamid = 1 : 1 : 1 oder 1 : 2 : 1 oder 1 :(1 bis 2) : 1 eingesetzt werden. Wird bei der Herstellung des Modifizierungsmittels ε-Caprolactam zu Formaldehyd zu Formamid = 1 : a : b eingesetzt und dabei mehr als 1 Mol Formamid verwendet, dann entstehen Gemische, in denen die Verbindungen der Formel Ia und/oder Ib neben anderen Umsetzungsprodukten, z. B. von Formamid und Formaldehyd bzw. ε-Caprolactam und Formaldehyd enthalten sind. Bei geeigneten Bedingungen kann das Gemisch beispielsweise auch Methylenbisformamid enthalten. Im Gegensatz zu Aminoplasten, die mit Methylenbisformamid modifiziert sind, behalten die mit den erfindungsgemäßen Verbindungen oder mit den genannten Gemischen modifizierten Aminoplaste ihre guten Eigenschaften während ihrer gesamten Lebensdauer bei. Die modifizierten Aminoplaste eignen sich auch ausgezeichnet als Kurztaktharze und sind darüber hinaus auch gegenüber Überhärtungen, wie sie in der Praxis z. B. durch verlängerte Pressenstandzeiten und/oder erhöhte Preßtemperaturen vorkommen können, weitgehend unempfindlich.

Die erreichte hohe Oberflächenelastizität der mit den genannten modifizierten Aminoplasten hergestellten Schichtstoffe und beschichteten Holzwerkstoffe ist z. B. dafür verantwortlich, daß bei der Prüfung auf Rißanfälligkeit gemäß DIN 53 799 vom Mai 1975, Ziffer 4.7.1 (Schichtstoffe), bzw. 4.7.2 (beschichtete Holzwerkstoffe), wobei die Proben 20 Stunden lang bei 80°C gelagert werden, keine Risse auftreten. (Diese Bestimmung der Rißanfälligkeit wird nachstehend Temperung bzw. Temperprüfung genannt.) Selbst bei einer Erhöhung der Temperatur auf 90°C treten in den meisten Fällen noch keine Risse auf. Die erfindungsgemäßen Kondensationsprodukte ε-Caprolactam zu Formaldehyd zu Formamid = 1 : a : b, wobei a eine Zahl von 1 bis 20, b eine Zahl von 1 bis 19 bedeutet und a und b so gewählt werden, daß der Quotient

$$\frac{a}{b+1} = 0{,}5 \text{ bis } 1$$

darstellt, sind wesentlich hydrolysestabiler als das Methylen-bis-formamid. Die erfrindungsgemäßen Kondensationsprodukte können in Form öliger Rohprodukte für die Modifizierung von Aminoplasten verwendet werden, was eine leichte Dosierung und Handhabung gestattet.

Sofern nichts anderes erwähnt, sind in den Beispielen angegebene Prozente Gewichtsprozente; Temperaturangaben sind in °Celsius gemacht.

## Beispiel 1

ε-Caprolactam zu Formaldehyd zu Formamid = 1 : 1 : 1

225 g Formamid, 2 g KOH und 170 g 90gew.-%iger Paraformaldehyd werden 10 Stunden lang bei 80°C gerührt. Dann werden 1,5 l Toluol, 560 g ε-Caprolactam und 6 g $KHSO_4$ hinzugefügt, und anschließend wird das Reaktionswasser (ca. 95 g) bei Normaldruck und einer Temperatur von 120°C abdestilliert. Die anfänglich ausgebildeten zwei Schichten gehen allmählich klar in Lösung. Wenn kein Wasser mehr übergeht, wird das als Schleppmittel verwendete Toluol abdestilliert. Es hinterbleiben 820 g (das sind 97% der Theorie) eines farblosen Öls, das mit Wasser in jedem Verhältnis mischbar ist und zur Modifizierung von Aminoplasten verwendet werden kann.

Aus dem Öl kann das reine N-(Formylaminomethyl)-ε-caprolactam durch Destillation in einem Dünnschichtverdampfer erhalten werden $Kp_{1,064 \text{ mbar}}$ = 180—184°C. Das reine N-(Formylaminomethyl)-ε-caprolactam kristallisiert in langen Kristallnadeln und besitzt einen Fp = 48°C.

Analyse:

| | | | |
|---|---|---|---|
| berechnet: | C 57,1 | H 7,1 | N 16,7 |
| gefunden: | C 57,3 | H 7,0 | N 16,4 |

Anstelle von Toluol kann auch ein anderes Schleppmittel verwendet werden, z. B. Xylol oder Äthylenchlorid.

## Beispiel 2

Herstellung einer Modifizierungskomponente mit einem Molverhältnis ε-Caprolactam zu Formaldehyd zu Formamid = 1 : 5 : 9

695 g Formamid, 195 g ε-Caprolactam, 285 g 90gew.-%iger Paraformaldehyd und 1 g KOH werden 10 Stunden lang bei 80°C gerührt, dann werden 4 g $KHSO_4$ zugegeben und bei 100°C und einem Druck von 266,6 mbar ca. 90 ml Reaktionswasser abdestilliert. Man erhält 950 g (80%) einer klaren viskosen Flüssigkeit, die in jedem Verhältnis mit Wasser mischbar ist und auch in unverdünntem Zustand nur wenig zur Kristallisation neigt.

## Beispiel 3

Herstellung einer Modifizierungskomponente mit einem Molverhältnis ε-Caprolactam zu Formaldehyd zu Formamid = 1 : 10 : 19

400 g Formamid, 60 g ε-Caprolactam, 165 g 90 gew.-%iger Paraformaldehyd und 10 g KOH werden 6 Stunden lang bei 80°C gerührt. Dann werden 2,5 g p-Toluolsulfonsäure und 600 ml Toluol zugegeben und das Wasser azeotrop abdestilliert. Wenn kein Wasser mehr übergeht, wird das Toluol abdestilliert, zuletzt im Wasserstrahlvakuum. Man erhält in praktisch quantitativer Ausbeute ein Öl, das in jedem Verhältnis mit Wasser mischbar ist.

Die azeotrope Abdestillation des Wassers kann umgangen werden, wenn das Gemisch nach dem Hinzufügen der 2,5 g p-Toluolsulfonsäure kontinuierlich in einen Dünnschichtverdampfer eingespeist wird, in dem es bei einer Manteltemperatur von 140°C und einem Druck von 333 mbar zur Reaktion gebracht und das Wasser laufend abdestilliert wird. Im Sumpf sammelt sich das gewünschte Reaktionsprodukt an. Auch bei dieser Verfahrensweise ist die Ausbeute praktisch quantitativ.

## Beispiel 4

3250 g 39gew.-%iger wäßriger Formaldehyd, 300 g Methanol, 250 g Wasser und 200 g einer 40gew.-%igen wäßrigen Lösung des Natriumsalzes der Amidosulfonsäure, 5 g Pottasche und 3100 g Melamin werden innerhalb von 30 bis 40 Minuten auf 90°C erhitzt und bei dieser Temperatur bis zu einer Wasserverdünnbarkeit von 1 : 2,2 kondensiert (Dauer ca. 4 Stunden). Der pH-Wert der Lösung soll 10,0 ± 0,2 betragen. Nach Abkühlen auf 50°C werden diesem »Grundharz« 400 g des nach Beispiel 2 hergestellten Kondensationsprodukts als Modifizierungsmittel zugemischt und mit Wasser auf einen Festkörpergehalt von 56 Gew.-% gestellt. Der Gehalt des Harzes an Modifizierungsmittel nach Beispiel 2 beträgt 8,4 Gew.-%, fest auf fest gerechnet.

4.1 Zu dieser Harzlösung wurde 0,9 Gew.-% Morpholinsalz der para-Toluolsulfonsäure als Härter (dieser Härter wird auch bei den folgenden Beispielen verwendet), bezogen auf Festharz, zugesetzt. In der mit Härter versetzten Tränkflotte wurde ein 80 g/m² schweres weißes Dekorpapier auf ein Endgewicht von ca. 200 g/m² imrpägniert und auf einen Restfeuchtegehalt von 5,5 bis 7 Gew.-% (5 Min./160°C) getrocknet.

(Die Angabe 5 Min/160°C besagt, daß zur Bestimmung des Restfeuchtegehalts eine

Probe 5 Minuten lang bei 160°C gelagert und aus dem dabei erlittenen Gewichtsverlust der Restfeuchtegehalt berechnet wurde.)

4.1.1) Ein Teil der Papiere wurde anschließend auf einer Kurztaktpresse mit einem Druck von 18 bis 22 bar bei einer Temperatur von 155°C auf Holzspanplatten aufgepreßt. Die Standzeit der Presse betrug 60 Sekunden. Die Oberflächen der Beschichtungen zeigten bei gleichmäßigem Glanz und guter Aushärtung nach einer Temperung von 20 Stunden bei 140°C keine Risse.

4.1.2) Ein Teil der imprägnierten Papiere wurde auf einer Kurztaktpresse mit einem Druck von 18 bis 22 bar bei einer Temperatur von 180°C und einer Standzeit von 3 Minuten auf Holzspanplatten aufgepreßt (Überhärtungspressung). Die Oberflächen der Beschichtungen zeigten bei gleichmäßigem Glanz und sehr hoher Aushärtung keine Risse.

4.1.3) Ein Teil der imprägnierten Papiere wurde auf einer Mehretagenpresse mit einem Druck von 18 bis 22 bar bei einer Temperatur von 140°C auf Holzspanplatten aufgepreßt. Die Standzeit der Presse betrug 10 Minuten. Danach wurde auf eine Temperatur von 70 bis 80°C zurückgekühlt und entformt. Die Oberflächen der beschichteten Holzspanplatten zeigten bei sehr guter Aushärtung gleichmäßigen Hochglanz und nach einer Temperung von 20 Stunden bei 80°C keine Risse.

4.2) Der Harzlösung des Beispiels 4 wurde 1,2 Gew.-% Härter, bezogen auf Festharz, zugesetzt. In der mit Härter versetzten Tränkflotte wurde ein 80 g/m² schweres Papier wie bei Beispiel 4.1 getränkt und getrocknet. Die imprägnierten Papiere wurden gemäß Beispiel 4.1.2 verpreßt. Die Oberflächen der Papiere zeigten bei gleichmäßigem Glanz und sehr hoher Aushärtung keine Risse.

4.3) Die Harzlösung des Beispiels 4 wurde ohne Härterzusatz 14 Tage bei Raumtemperatur (20 bis 25°C) gelagert, danach mit 0,9 Gew.-% Härter, bezogen auf Festharz, versetzt. In dieser Lösung wurde gemäß Beispiel 4.1 ein 80 g/m² schweres, weißes Papier imprägniert und wie Beispiel 4.1.2 verpreßt. Die Oberflächen der Beschichtungen blieben bei gleichmäßigem Glanz und sehr hoher Aushärtung rißfrei.

4.4) Die Harzlösung des Beispiel 4 wurde mit 0,4 Gew.-% Härter versetzt. Nach Verdünnen mit Wasser auf eine Konzentration von ca. 52 Gew.-% wurde in der Lösung ein ca. 30 g/m² schweres, aus $\alpha$-Cellulose bestehendes, hochtransparentes Overlay-Papier auf ein Endgewicht von 100 bis 105 g/m² imprägniert und auf eine Restfeuchte von 6,5 bis 7 Gew.-% getrocknet, und ein 120 g/m² schweres Dekorpapier wurde auf ein Endgewicht von 200 bis 210 g/m² imprägniert und einen Restfeuchtegehalt von 5,5 Gew.-% getrocknet. Zusammen mit in Phenolharz getränkten Natronkraftpapieren wurde ein Schichtstoff hergestellt.

Aufbau:
1 Overlay-Papier
1 Dekorpapier
9 phenolharzimprägnierte Kraftpapiere
1 Trennfolie (silikonisiertes Papier).

Gepreßt wurde 8 Minuten bei 140°C und einem Druck von 80 bar. Vor dem Entformen wurde auf 70 bis 80°C zurückgekühlt. Die hergestellte Schichtstoffplatte zeigte nach der Temperung, die 20 Stunden lang bei 80°C geführt wurde, keine Rißbildung.

4.5) Vergleichsbeispiel
In das in Beispiel 4 beschriebene Grundharz werden 400 g Methylenbisformamid gemäß DE-A-2 149 970 eingemischt und sonst wie in Beispiel 4 verfahren. Zur erhaltenen Harzlösung wurde 0,9 Gew.-% Härter, bezogen auf Festharz, zugesetzt. In dieser Tränkflotte wurde ein 80 g schweres Papier gemäß Beispiel 4.1 imprägniert, getrocknet und gemäß Beispiel 4.1.2 verpreßt. Die Oberflächen der Beschichtungen zeigten bei gleichmäßigem Glanz und sehr hoher Aushärtung keine Risse.
Die Harzlösung wurde ohne Härterzusatz 14 Tage bei Raumtemperatur gelagert und wie oben verarbeitet. Die Oberflächen der Beschichtungen zeigten einzelne Risse über den ganzen Flächen.

4.6) Vergleichsbeispiel
Die Herstellung der Harzlösung gemäß Beispiel 4 wurde mit der Abänderung wiederholt, daß das Modifizierungsprodukt weggelassen wurde. Unter Verwendung der so hergestellten Aminoharzlösung wurden wie in Beispiel 4.1.2 Holzspanplatten beschichtet. Die Oberflächen der beschichteten Spanplatten zeigen Rißbildungen über die ganze Fläche.

## Beispiel 5

In das Grundharz gemäß Beispiel 4 werden 490 g des Modifizierungsmittels des Beispiels 2 eingemischt (entspricht ca. 10 Gew.-% fest auf fest gerechnet). Alle sonstigen Reaktionsbedingungen werden konstant gehalten. Imprägniert wurde wie folgt:

5.1.1) Der Harzlösung von Beispiel 5 wurden 1,2 Gew.-% Härter, auf Festharz bezogen, zugesetzt. In dieser Lösung wurde ein 80 g schweres Papier gemäß Beispiel 4.1 imprägniert und ein Teil der Papiere wie in Bespiel 4.1.2 auf Holzspanplatten aufgepreßt. Die Oberflächen der Beschichtungen zeigten bei gleichmäßigem Glanz und

sehr hoher Aushärtung über den gesamten Restfeuchtebereich von 5 bis 8 Gew.-% keine Risse.

5.1.2) Ein zweiter Teil der Papiere wurde auf einer Kurztaktpresse bei 180° C und einem Druck von 18 bis 22 bar und einer Standzeit von 5 Minuten aufgepreßt. Auch hier zeigten die Oberflächen der Beschichtungen über einen Restfeuchtebereich von 5 bis 8 Gew.-% bei gleichmäßigem Glanz und sehr hoher Aushärtung keine Rißbildung.

5.2) Der Harzlösung von Beispiel 5 wurden 0,9% Härter zugesetzt. In dieser Tränkflotte wurde ein 80 g/m² schweres weißes Papier gemäß Beispiel 4.1 imprägniert, getrocknet und wie Beispiel 4.1.3 auf Holzspanplatten aufgepreßt. Die Oberflächen der beschichteten Holzspanplatten zeigten bei sehr guter Aushärtung und gleichmäßigem Hochglanz nach Temperung von 20 Stunden bei 80° C keine Risse.

Beispiel 6

In das Grundharz gemäß Beispiel 4 werden 290 g des Modifizierungsmittels nach Beispiel 2 eingemischt (ca. 6 Gew.-% fest auf fest gerechnet). Alle sonstigen Reaktionsbedingungen werden konstant gehalten.

4.1) Der Harzlösung von Beispiel 6 wurde 0,9% Härter zugesetzt und weiter verarbeitet wie unter Beispiel 4.1.2. Die Oberflächen der so beschichteten Spanplatten blieben im Restfeuchtebereich von 6 bis 7 Gew.-% rißfrei.

4.2) Der Harzlösung von Beispiel 6 wurde 0,4% Härter zugesetzt und weiterverarbeitet gemäß Beispiel 4.1.3. Auch die so hergestellten dekorativ beschichteten Holzspanplatten zeigten bei hohem Glanz und guter Aushärtung nach Temperung bei 20 Stunden bei 80° C keine Risse.

Beispiel 7

In das Grundharz gemäß Beispiel 4 werden 145 g des gemäß Beispiel 2 beschriebenen Modifizierungsmittels (ca. 3 Gew.-% fest auf fest gerechnet) und 70 g Diäthylenglykol zugemischt. Alle sonstigen Bedingungen des Beispiels 4 bleiben konstant.

7.1) Die Harzlösung von Beispiel 7 wurde mit 0,4% Härter, bezogen auf Festharz, versetzt und weiterverarbeitet gemäß Beispiel 4.1.3. Die Oberflächen der so beschichteten Holzspanplatten zeigen bei guter Aushärtung Hochglanz und bleiben auch nach einer Temperung von 20 Stunden bei 80° C rißfrei.

Beispiel 8

380 g wäßrige, 39gew.-% Formaldehydlösung, 10 g Äthylenglykol, 20 g Methanol, 10 g Zucker, 6 g einer 40gew.-%igen Lösung des Natriumsalzes der Amidosulfonsäure werden auf einen pH-Wert von 10,1 gestellt und unter Rühren 330 g Melamin zugegeben. Dieses Gemisch wird auf 90° C erhitzt und bis zu einer Wasserverdünnbarkeit von 1 : 1,5 kondensiert. Nach Abkühlung auf 50° C werden 40 g des Modifizierungsproduktes gemäß Beispiel 2 und 150 g Wasser zugegeben. Der Gehalt der Harzlösung an Modifizierungsmittel gemäß Beispiel 2 beträgt 5,3 Gew.-%, bezogen auf Festharz. Diese Harzlösung wird mit 0,4% Härter, bezogen auf Festharz, versetzt. In dieser Lösung wird ein 80 g/m² schweres Dekorpapier wie unter 4.1 imprägniert und gemäß 4.1.3 auf Holzspanplatten aufgepreßt. Die Oberflächen der so beschichteten Spanplatten zeigen bei guter Aushärtung gleichmäßigen hohen Glanz und bleiben nach 20stündiger Temperung bei 80° C rißfrei.

Beispiel 9

36,0 kg wäßrige, 39gew.-%ige Formaldehydlösung, 2,7 kg Diäthylenglykol, 1,0 kg Methanol, 2,2 kg Natriumsalz der Amidosulfonsäure, 3,7 kg Zucker und 15,0 kg Wasser werden auf einen pH-Wert von 10,1 mit Dimethylaminoäthanol gestellt und 32 kg Melamin zugegeben. Dieses Gemisch wird bei 90° C auf eine Wasserverdünnbarkeit von 1 : 2,0 kondensiert, auf 50° C abgekühlt und 3 kg des Modifizierungsmittels gemäß Beispiel 2 sowie 5 kg Wasser zugefügt. Der Gehalt der Harzlösung, bezogen auf Festharz, an Modifizierungsmittel gemäß Beispiel 2 beträgt 5,6 Gew.-%.

9.1) Dieser Harzlösung wurden 0,9 Gew.-% Härter, bezogen auf Festharz zugesetzt. In der so erhaltenen Harzlösung wird ein 80 g/m² schweres Papier gemäß 4.1 imprägniert und mit dem imprägnierten Papier Holzspanplatten wie in 4.1.2 beschichtet. Die Oberflächen der beschichteten Platten bleiben bei gleichmäßigem Glanz und sehr hoher Aushärtung rißfrei.

9.2) Die Harzlösung von Beispiel 9 wurde mit 0,4 Gew.-% Härter, bezogen auf Festharz, versetzt und gemäß Beispiel 4.1.3 weiterverarbeitet. Die Oberflächen der so beschichteten Holzspanplatten zeigen bei guter Aushärtung gleichmäßigen Hochglanz und bleiben nach 20stündiger Temperung bei 80° C rißfrei.

Beispiel 10

3200 g Formaldehyd (39gew.-%ige wäßrige Lösung), 400 g Wasser und 200 g einer 40gew.-%igen wäßrigen Lösung des Natriumsalzes der

Amidosulfonsäure werden mit 2 N NaOH auf einen pH-Wert von 10,2 gestellt und 3100 g Melamin und 100 g des in Beispiel 2 beschriebenen Modifizierungsproduktes . zugefügt. Dann wird bei 90° C bis auf eine Wasserverdünnbarkeit von 1 : 4 kondensiert und weitere 200 g des Modifizierungsproduktes zugefügt. Man kondensiert bei 90° C bis auf eine Wasserverdünnbarkeit von 1 : 1,5, kühlt auf 50° C ab und gibt erneut 150 g des Modifizierungsproduktes zu. Dieses Harz wird sprühgetrocknet und ist so unbegrenzt lagerfähig. Die anwendungstechnischen Eigenschaften sind wie in Beispiel 5 beschrieben.

Beispiel 11

500 g des in Beispiel 1 erhaltenen Rohprodukts werden mit 100 g 90gew.-%igem Paraformaldehyd und 0,4 g Pottasche 10 Stunden bei 80° C gerührt. Es wird ein Öl erhalten, das auch nach längerer Zeit nicht erstarrt und sehr gut handhabbar ist. Die Ausbeute ist praktisch quantitativ.

Analyse:

| | | | |
|---|---|---|---|
| berechnet: | C 54,0 | H 8,0 | N 14,0 |
| gefunden: | C 53,8 | H 8,2 | N 14,2 |

Brechnungsindex: 1,4995 bei 20° C

Beispiel 12

450 g Formamid, 630 g 90gew.-%iger Paraformaldehyd und 2 g KOH werden bei 80° C fünf Stunden lang gerührt. Zu der erhaltenen Schmelze von Dimethylolformamid werden 1130 g ε-Caprolactam und 8 g Kaliumhydrogensulfat hinzugefügt. In einem Vakuum von 266 mbar wird unter Rühren das Reaktionswasser bei einer Innentemperatur von 120° C ± 10° C abdestilliert. Das erhaltene Produkt ist mit dem Produkt nach Beispiel 11 identisch.

**Patentansprüche**

1. Kondensationsprodukt aus ε-Caprolactam, Formaldehyd und Formamid im Molverhältnis 1 : a : b, wobei a eine Zahl von 1 bis 20, b eine Zahl von 1 bis 19 bedeutet und a und b so gewählt werden, daß der Quotient

$$\frac{a}{b+1} = 0{,}5 \text{ bis } 1$$

darstellt, erhalten durch Erhitzen von Formamid und Formaldehyd in Gegenwart einer Base und gegebenenfalls von ε-Caprolactam bei Temperaturen von 70 bis 90° C während 4 bis 15 Stunden, Sauerstellen des Reaktionsansatzes und, sofern noch kein ε-Caprolactam vorhanden ist, Hinzufügen von ε-Caprolactam und Abdestillieren von Wasser bei Temperaturen von 100 bis 135° C.

2. N-(Formylaminomethyl)-ε-caprolactam.

3. N-(N′-Formyl-N′-hydroxymethyl-amino methyl)-ε-caprolactam.

4. Modifizierter Aminoplast, gekennzeichnet durch einen Gehalt von 0,5 bis 40 Gew.-%, bezogen auf den Festkörpergehalt des fertigen Harzes, an einem Kondensationsprodukt aus ε-Caprolactam, Formaldehyd und Formamid nach Anspruch 1 bis 3 als Modifizierungsmittel.

5. Modifizierter Aminoplast nach Anspruch 4, gekennzeichnet durch einen Gehalt von 2,5 bis 15 Gew.-% des Modifizierungsmittels.

6. Verfahren zur Herstellung des Aminoplasten nach den Ansprüchen 4 bis 5, dadurch gekennzeichnet, daß Aminoplastbildner und Carbonylverbindung und gegebenenfalls Modifizierungsmittel in an sich bekannter Weise bis zu einer begrenzten oder unbegrenzten Wasserverdünnbarkeit kondensiert werden und vor, während oder nach der Kondensation als Modifizierungsmittel ein Kondensationsprodukt aus ε-Caprolactam, Formaldehyd und Formamid nach Anspruch 1 bis 3 in einer solchen Menge zugefügt wird, daß der fertige Aminoplast davon, bezogen auf den Festkörpergehalt des fertigen Harzes, den in Anspruch 4 bzw. 5 angegebenen Gehalt besitzt.

**Claims**

1. Condensation produkt of ε-caprolactam, formaldehyde and formamide in a molar ratio of 1 : a : b, where a is a number from 1 to 20, b is a number from 1 to 19 with the provise that the quotient of

$$\frac{a}{b+1} \text{ is } 0.5 \text{ to } 1,$$

obtained by heating formamide and formaldehyde in the presence of a base and optionally of ε-caprolactam at temperatures of 70 to 90° C for a 4 to 15 hours, acidifying the reaction batch, adding ε-caprolactam, if not previously present, and distilling off water at temperatures of 100 to 135° C.

2. N-(Formylaminomethyl)-ε-caprolactam.

3. N-(N′-Formyl-N′-hydroxymethyl-aminomethyl)-ε-caprolactam.

4. Modified aminoplast characterised by a content of 0.5 to 40% by weight, relative to the solids content of the finished resin, of a condensation produkt of ε-caprolactam, formaldehyde and formamide according to claims 1 to 3 as modifying agent.

5. Modified aminoplast according to claim 4, characterised by a content of 2.5 to 15% by weight of the Modifying agent.

6. Process for preparing the aminoplast according to claims 4 and 5, chracterised in that the aminoplast precursor and the carbonyl compound and optionally the modifying agent are condensed in a manner known per se until a

limited or unlimited water-dilutability is reached and that before, during or after the condensation a condensation product of ε-caprolactam, formaldehyde and formamide according to claims 1 to 3 is added as modifying agent in such an amount that the finished aminoplast has the content thereof, indicated in claims 4 and 5 respectively, relative to the solids content of the finished resin.

**Revendications**

1. Produit de condensation de l'ε-caprolactame, du formaldéhyde et du formamide dans la proportion molaire 1 : a : b, la lettre a désignant un nombre de 1 à 20, b un nombre de 1 à 19 et a et b étant choisis de telle façon que le quotient

$$\frac{a}{b+1} \text{ soit compris entre 0,5 et 1,}$$

produit obtenu par chauffage du formamide et du formaldéhyde en présence d'une base et éventuellement d'ε-caprolactame, à des températures de 70 à 90°C, pendant 4 à 15 heures, acidification du mélange réactionnel et, lorsqu'il n'y a pas encore d'ε-caprolactame, addition d'ε-caprolactame et élimination de l'eau par distillation à des températures de 100 à 135° C.

2. N-(Formylaminométhyl)-ε-caprolactame.

3. N-(N'-Formyl-N'-hydroxyméthyl-amino méthyl)-ε-caprolactame.

4. Aminoplaste modifié, caractérisé en ce qu'il contient, comme agent modificateur, de 0,5 à 40% en poids, par rapport à la teneur en matières solides de la résine finie, d'un produit de condensation d l'ε-caprolactame, du formaldéhyde et du formamide selon l'une quelconque des rèvendications 1 à 3.

5. Aminoplaste modifié selon la revendication 4, caractérisé en ce qu'il contient de 2,5 à 15% en poids de l'agent modificateur.

6. Procédé de préparation de l'aminoplaste selon l'une des rèvendications 4 et 5, caractérisé en ce qu'on condense un formateur d'aminoplaste, un composé carbonylique et, éventuellement, un agent modificateur, de manière connue, jusqu'à ce qu'on ait atteint une diluabilité à l'eau limitée on infinie, et, avant, pendant ou après la condensation, on ajoute, comme agent modificateur, un produit de condensation de l'ε-caprolactame, du formaldéhyde at du formamide selon l'une quelconque des rèvendications 1 à 3, en une quantité telle que l'aminoplaste fini présente la teneur en cet agent modificateur, par rapport à la teneur en matières solides de la résine finie, qui est indiquée à l'une des revendications 4 et 5.